# FASCICULE DE BREVET EUROPEEN

(11) **EP 1 150 961 B1**
(45) Date de publication et mention de la délivrance du brevet: **22.10.2003**
(21) Numéro de dépôt: 00901661.9
(22) Date de dépôt: 28.01.2000
(51) Int. Cl.: C07D 231/14, A61K 31/415, A61P 25/00, C07C 69/738, C07C 251/80

(54) **DERIVES D'ACIDE PYRAZOLECARBOXYLIQUE, LEUR PREPARATION, LES COMPOSITIONS PHARMACEUTIQUES EN CONTENANT**
PYRAZOLCARBONSÄUREDERIVATE, VERFAHREN ZU IHRER HERSTELLUNG UND DIESE ENTHALTENDE PHARMAZEUTISCHE ZUSAMMENSETZUNGEN
PYRAZOLECARBOXYLIC ACID DERIVATIVES, THEIR PREPARATION, PHARMACEUTICAL COMPOSITIONS CONTAINING THEM

(30) Priorité: 01.02.1999 FR 9901201; 02.08.1999 FR 9910166
(43) Date de publication de la demande: 07.11.2001
(73) Titulaire: SANOFI-SYNTHELABO, 75013 Paris (FR)
(72) Inventeur: CAMUS, Philippe, F-31600 Muret (FR); MARTINEZ, Serge, F-34000 Montpellier (FR); RINALDI, Murielle, F-34680 St. Georges d'Orques (FR); Barth, Francis, 34070 Montpellier (FR)
(74) Mandataire: Ludwig, Jacques
(86) Numéro de dépôt international: FR0000194
(87) Numéro de publication internationale: WO00046209

(56) Documents cités:
- EP-A- 0 576 357
- EP-A- 0 656 354
- DATABASE CHEMABS [Online] CHEMICAL ABSTRACTS SERVICE, COLUMBUS, OHIO, US THOMAS, BRIAN F. ET AL: "Comparative receptor binding analyses of cannabinoid agonists and antagonists" retrieved from STN Database accession no. 129:12298 XP002136951 & J. PHARMACOL. EXP. THER. (1998), 285(1), 285-292 ,
- DATABASE CHEMABS [Online] CHEMICAL ABSTRACTS SERVICE, COLUMBUS, OHIO, US LAN, RUOXI ET AL: "Preparation of iodine-123 labeled AM251: a potential SPECT radioligand for the brain cannabinoid CB1 receptor" retrieved from STN Database accession no. 125:321765 XP002136952 & J. LABELLED COMPD. RADIOPHARM. (1996), 38(10), 875-881 ,

## Description

La présente invention concerne un nouveau dérivé du pyrazole, ses sels et leurs solvats, un procédé pour leur préparation et des compositions pharmaceutiques les contenant.

Les demandes de brevet EP-A-576 357, EP-A-658 546 et

WO-97/19063 décrivent des dérivés du pyrazole présentant une affinité pour les récepteurs aux cannabinoïdes. Plus particulièrement, la demande de brevet EP-A-656 354 décrit le N-pipéridino-5-(4-chlorophényl)-1-(2,4-dichlorophényl)-4-méthylpyrazole-3-carboxamide, également dénommé SR 141 716, et ses sels pharmaceutiquement acceptables qui présentent une très bonne affinité pour les récepteurs aux cannabinoïdes centraux.

Des composés proches du SR 141716 ont été décrits dans la littérature, notamment le N-pipéridino-5-(4-bromophényl)-1-(2,4-dichlorophényl)-4-méthyl pyrazole-3-carboxamide, ci-après dénommé composé A, qui est décrit par B.F. Thomas et al. dans J. Pharm. Exp. Therap., 1998, 285, 285-292.

Les effets des cannabinoïdes sont dûs à une intéraction avec des récepteurs spécifiques de haute affinité présents au niveau central (Devane et al., Mol. Pharmacol., 1988, 34, 605-613) et périphérique (Nye et al., Pharmacol. and Experimental Ther., 1985, 234, 784-791 ; Kaminski et al., 1992, Mol. Pharmacol., 42, 736-742 ; Munro et al., Nature, 1993, 365, 61-65).

La caractérisation des récepteurs a été rendue possible par la mise au point de ligands synthétiques spécifiques des récepteurs aux cannabinoïdes tels que les agonistes WIN 55212-2 (J. Pharmacol. Exp. Ther., 1993, 264, 1352-1363) ou le CP 55,940 (J. Pharmacol. Exp. Ther., 1988, 247, 1046-1051). La pharmacologie des sous-types CB₁ et CB₂ des récepteurs aux cannabinoïdes est exposée dans Pharmacol. Ther., 1997, 74, 129-130.

On a maintenant trouvé un nouveau dérivé de N-pipéridino-3-pyrazole carboxamide qui possède une très bonne affinité pour le sous-type CB₁ des récepteurs aux cannabinoïdes (récepteurs CB₁) avec une longue durée d'action et qui est utile dans les domaines thérapeutiques où les cannabinoïdes sont connus pour intervenir.

Selon un de ses aspects, la présente invention concerne le N-pipéridino-5-(4-bromophényl)-1-(2,4-dichloro phényl)-4-éthylpyrazole-3-carboxamide de formule : 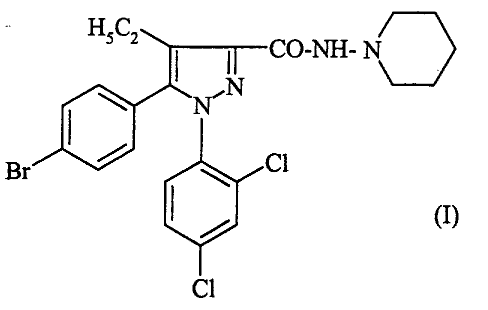 ses sels pharmaceutiquement acceptables et leurs solvats.

Selon un autre de ses aspects, la présente invention concerne un procédé pour la préparation du composé (I) ci-dessus, de ses sels et de leurs solvats, caractérisé en ce qu'on traite un dérivé fonctionnel de l'acide 5-(4-bromophényl)-1-(2,4-dichlorophényl)-4-éthylpyrazole-3-carboxylique de formule : 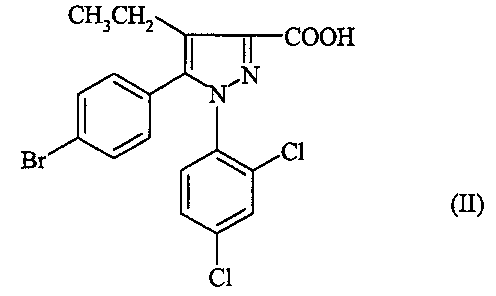 avec la 1-aminopipéridine, dans un solvant organique et en présence d'une base ; et éventuellement on transforme le composé ainsi obtenu en l'un de ses sels ou l'un de leurs solvats.

La réaction est effectuée en milieu basique, par exemple en présence de triéthylamine dans un solvant inerte tel que le dichlorométhane ou le tétrahydrofurane.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de p-nitrophényle, ou l'acide libre opportunément activé, par exemple, avec le N,N-dicyclohexylcarbodiimide ou avec l'hexafluorophosphate de benzotriazol-N-oxotris(diméthylamino)phosphonium (BOP).

Ainsi par le procédé selon l'invention, on peut faire réagir le chlorure de l'acide de formule (II) obtenu par réaction du chlorure de thionyle sur l'acide de formule (II) dans un solvant inerte, tel que le benzène ou le toluène, ou un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température de reflux du solvant.

Une variante au mode opératoire consiste à préprarer l'anhydride mixte de l'acide de formule (II) par réaction du chloroformiate d'éthyle avec l'acide de formule (II), en présence d'une base telle que la triéthylamine.

L'acide de formule (II) peut être préparé selon le Schéma réactionnel décrit ci-après dans lequel :
LiHMDS : sel de lithium de l'hexaméthyldisilazane
NBS : N-bromosuccinimide.

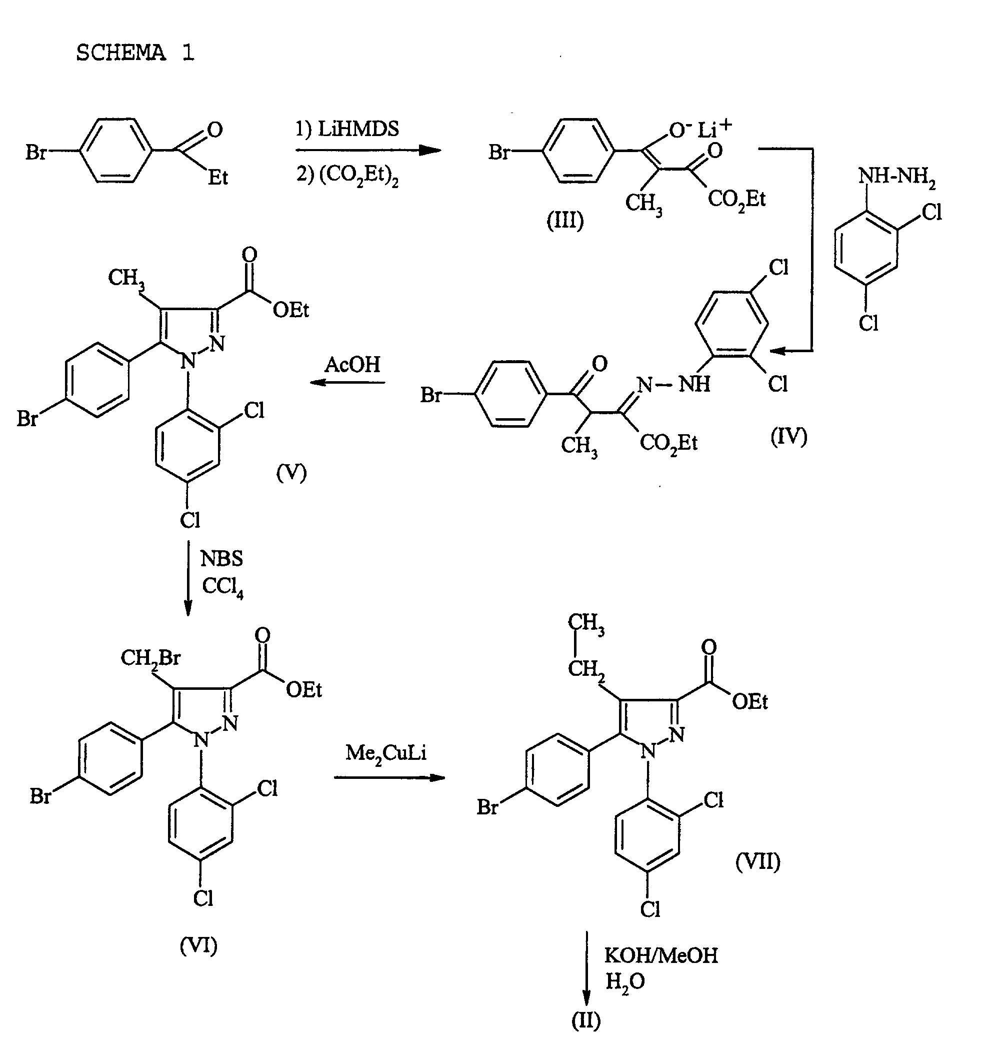

La première étape est effectuée d'après J. Heterocyclic. Chem., 1989, 26, 1389. A l'avant-dernière étape, la transformation du substituant 4-bromométhyle du pyrazole en 4-éthyle est effectuée selon J. Am. Chem. Soc., 1968, 90, 5615.

La 1-aminopipéridine utilisée est un produit commercial.

L'ester de formule (VII) et l'acide de formule (II) peuvent être préparés selon un autre procédé qui constitue un objet ultérieur de la présente ivention.

Ce procédé est illustré par le schéma réactionnel ci-après dans lequel Alk représente un (C₁-C₆)alkyle et représente un éthyle.

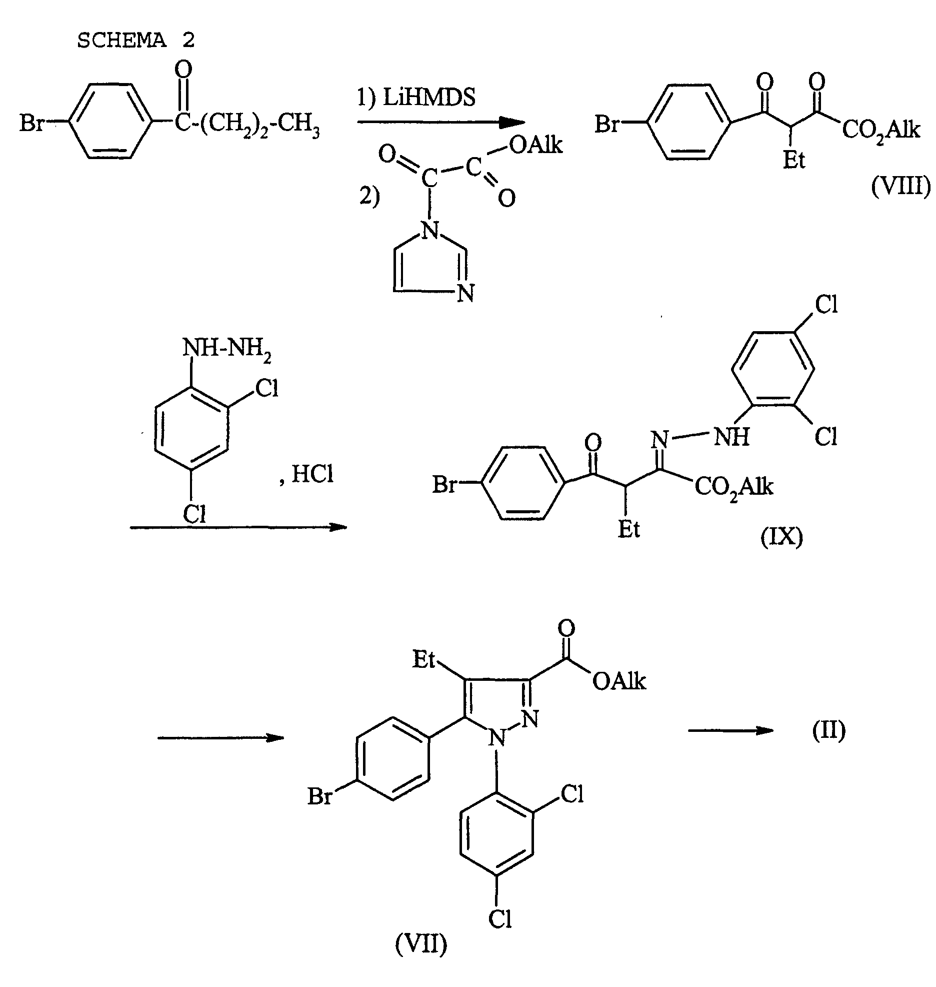

Ce procédé est caractérisé en ce qu'un ester alkylique, préférentiellement éthylique, de l'acide 5-(4-bromophényl)-1-(2,4-dichlorophényl)-4-éthylpyrazole-3-carboxylique est préparé par cyclisation d'un ester alkylique, préférentiellement éthylique, de l'acide 3-(4-bromobenzoyl)-2-(2-(2,4-dichlorophényl)hydrazono) pentanoate (IX).

Cette réaction est effectuée dans un solvant protique tel qu'un alcool, par exemple un alcool en C₁-C₄, préférentiellement l'éthanol, à une température comprise entre la température ambiante et 80°C, préférentiellement à reflux de l'éthanol.

Selon l'invention, l'ester alkylique, préférentiellement éthylique, de l'acide 3-(4-bromobenzoyl)-2-(2-(2,4-dichlorophényl)hydrazono)pentanoate est préparé par action d'un sel de 2,4-dichlorophénylhydrazine, préférentiellement le chlorhydrate, sur un ester alkylique, préférentiellement éthylique, de l'acide 4-bromobenzoyl-2-oxopentanoïque (VIII).

La réaction est effectuée dans un solvant protique, par exemple un alcool en C₁-C₄, préférentiellement l'éthanol.

Selon l'invention l'ester alkylique, préférentiellement éthylique, de l'acide 4-bromobenzoyl-2-oxopentanoïque est préparé par action de LiHMDS puis d'un ester alkylique, préférentiellement éthylique, de l'acide 2-(imidazol-1-yl)-2-oxoacétique sur la bromobutyrophénone.

La réaction est effectuée dans un solvant organique tel qu'un solvant aromatique ou un éther, préférentiellement le méthyl*-tert*-butyléther. La première étape de cette réaction est effectuée à basse température, par exemple à une température comprise entre 0°C et -60°C, préférentiellement à une température voisine de -20°C ; la seconde étape est réalisée à une température comprise entre la température ambiante et -20°C, préférentiellement à température ambiante.

Ainsi selon le schéma 2, la préparation d'un ester alkylique de l'acide 5-(4-bromophényl)-1-(2,4-dichlorophényl)-4-éthylpyrazole-3-carboxylique (VII) est effectuée à partir de l'acide 4-bromobenzoyl-2-oxopentanoïque (VIII) par action d'un sel de 2,4-dichlorophénylhydrazine puis cyclisation.

La bromobutyrophénone est commerciale.

L'ester éthylique de l'acide 2-(imidazol-1-yl)-2-oxoacétique est décrit et préparé selon J. Org.. Chem., 1981, 46 (1), 211-213.

La présente invention comprend également un procédé de préparation d'un ester alkylique, préférentiellement éthylique, de l'acide 5-(4-bromophényl)-1-(2,4-dichlorophényl)-4-éthylpyrazole-3-carboxylique à partir de l'acide 4-bromobenzoyl-2-oxopentanoïque par action d'un sel de 2,4-dichlorophénylhydrazine, préférentiellement le chlorhydrate, dans un solvant protique, par exemple un alcool en C₁-C₄, préférentiellement l'éthanol. La réaction est effectuée à une température comprise entre la température ambiante et 80°C, préférentiellement à reflux de l'éthanol.

Les composés de formule : 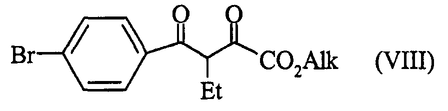 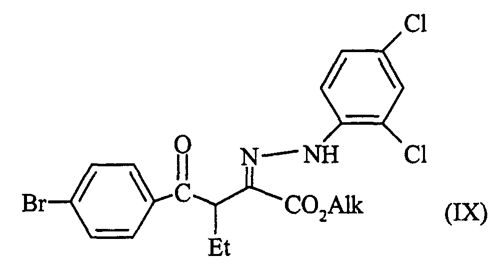 dans lesquels Alk représente un (C₁-C₆)alkyle sont nouveaux et font partie de l'invention. Préférentiellement Alk représente un éthyle.

Le composé de formule (I) obtenu par le procédé selon l'invention est isolé, sous forme de base libre ou de sel ou de solvat, selon les techniques conventionnelles.

Les sels pharmaceutiquement acceptables du composé de formule (I) comprennent les sels d'addition d'acides tels que le chlorhydrate, le bromhydrate, le sulfate, l'hydrogénosulfate, le dihydrogénophosphate, le méthanesulfonate, le méthylsulfate, l'oxalate, le maléate, le fumarate, le 2-naphtalènesulfonate, le glyconate, le gluconate, le citrate, l'iséthionate, le paratoluènesulfonate, le succinate.

Le composé de formule (I) peut être isolé sous forme d'un de ses sels, par exemple le chlorhydrate ou l'oxalate ; dans ce cas, la base libre peut être préparée par neutralisation dudit sel avec une base minérale ou organique, telle que l'hydroxyde de sodium ou d'ammonium, la triéthylamine ou un carbonate ou bicarbonate alcalin tel que le carbonate ou le bicarbonate de sodium ou de potassium, et transformée en un autre sel comme le méthanesulfonate, le fumarate ou le 2-naphtalènesulfonate.

Lorsque le composé de formule (I) est obtenu sous forme de base libre, la salification est effectuée par traitement avec l'acide choisi dans un solvant organique. Par traitement de la base libre, dissoute par exemple dans un éther tel que l'éther diéthylique ou dans l'acétone, avec une solution de l'acide dans le même solvant, on obtient le sel correspondant qui est isolé selon les techniques classiques.

Les composés de formule (I) possèdent une très bonne affinité *in vitro* pour les récepteurs aux cannabinoïdes CB₁, dans les conditions expérimentales décrites par Devane et al., Mol. Pharmacol., 1988, 34, 605-613.

Ainsi le composé selon l'invention présente une très forte affinité pour les récepteurs aux cannabinoïdes CB₁ humains (Ki = 5,4 nM) qui se compare favorablement à celle du SR 141716 pour les mêmes récepteurs, déterminée dans les mêmes conditions (Ki = 34 nM).

Le composé selon l'invention a également été comparé au N-pipéridino-5-(4-bromophényl)-1-(2,4-dichlorophényl)-4-méthylpyrazole-3-carboxamide, (composé A). L'affinité pour les récepteurs aux cannabinoïdes CB₁ humains, de ce composé, mesurée dans les mêmes conditions, se traduit par un Ki de 8 nM.

Par ailleurs, on a comparé la durée d'occupation des récepteurs CB₁ présents dans le cerveau par les 3 composés suivants :
- le composé de formule (I) selon l'invention,
- le SR 141716,
- le composé A.

L'étude a été réalisée *in vivo* chez la souris, après administration orale de chacun des composés à la dose de 10 mg/kg, selon la technique décrite dans M.

Rinaldi-Carmona et al., Life Sciences, 1995, 56, 1941-1947. Les résultats obtenus sont rassemblés dans le tableau ci-dessous.

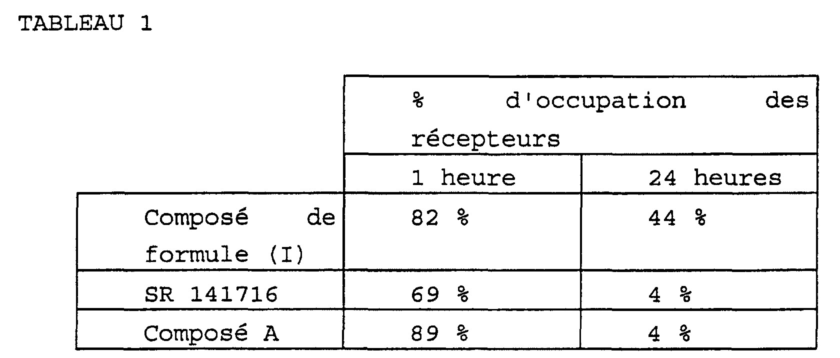

De façon surprenante, on observe que le composé de formule (I) selon l'invention est le seul à présenter une occupation importante (44 %) 24 heures après son administration.

D'autre part, la nature antagoniste du composé de formule (I) a été démontrée par les résultats obtenus dans les modèles de l'inhibition de l'adénylate-cyclase comme décrits dans M. Rinaldi-Carmona et al., J. Pharmacol. Exp. Ther., 1996, 278, 871-878.

Plus particulièrement, le composé de la présente invention, tel quel ou sous forme d'un de ses sels pharmaceutiquement acceptables, est un antagoniste puissant et sélectif des récepteurs aux cannabinoïdes CB₁.

La nature antagoniste du composé selon l'invention ainsi que sa bonne pénétration dans le système nerveux central sont confirmées par les résultats obtenus dans le modèle de l'antagonisme de l'hypothermie provoquée par un agoniste des récepteurs aux cannabonoïdes. Ainsi le composé de formule (I) selon l'invention antagonise l'hypothermie induite par le WIN 55212-2 chez la souris avec une DE₅₀ 0,3 mg/kg *per os* dans le test décrit par Pertwee R.G. et al. dans Marijuana, 84, Ed. Harvey, D.Y. Oxford IRL Press, 1985, 263-277. Dans ce test, on a comparé l'activité et la durée d'action de 3 composés. Les résultats obtenus sont rassemblés dans le tableau ci-après :

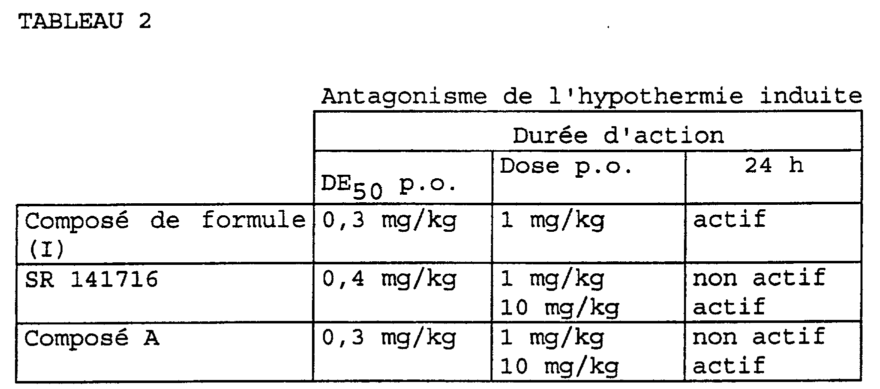

On constate que le composé de la présente invention présente une DE₅₀ comparable à celles des composés de l'art antérieur, cependant que sa durée d'action est nettement supérieure.

Ainsi, alors que 24 heures après leur administration le SR 141716 et le composé A ne sont actifs qu'à la dose de 10 mg/kg/p.o., le composé de formule (I) selon l'invention, est actif 24 heures après son administration, à une dose 10 fois inférieure (1mg/kg/p.o.).

La longue durée d'action du composé de formule (I) selon l'invention est particulièrement remarquable et représente un avantage important pour son utilisation en tant que médicament.

La toxicité des composés (I) est compatible avec leur utilisation en tant que médicament.

Selon un autre de ses aspects, la présente invention concerne l'utilisation d'un composé de formule (I), ou de l'un de ses sels ou solvats pharmaceutiquement acceptable, pour la préparation de médicaments destinés à traiter les maladies impliquant les récepteurs aux cannabinoïdes CB₁.

Par exemple et de manière non limitative, le composé de formule (I) est utile comme médicament psychotrope, notamment pour le traitement des troubles anxieux, des troubles de l'humeur, des troubles délirants, des troubles psychotiques en général, pour le traitement de la schizophrénie, de la dépression, ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Le composé de formule (I) selon l'invention peut être utilisé comme médicament pour le traitement des neuropathies, de la migraine, du stress, des maladies d'origine psychosomatique, de l'épilepsie, des troubles du mouvement, en particulier des dyskinésies ou de la maladie de Parkinson.

Le composé de formule (I) selon l'invention peut également être utilisé comme médicament dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, le composé de formule (I) peut être utile comme neuroprotecteur, dans le traitement des maladies neurodégénératives.

Le composé de formule (I) selon l'invention peut être utilisé comme médicament dans le traitement des troubles de l'appétit, de l'appétence (pour les sucres, carbohydrates, drogues, alcools ou toute substance appétissante) et/ou des conduites alimentaires, notamment en tant qu'anorexigène ou pour le traitement de l'obésité ou de la boulimie ainsi que pour le traitement du diabète de type II ou diabète non insulinodépendant. De plus, le composé de formule (I) selon l'invention peut être utilisé en tant que médicament dans le traitement des troubles gastro-intestinaux,des troubles diarrhéiques, des ulcères, des vomissements, des troubles vésicaux et urinaires, des troubles cardio-vasculaires, des troubles de la fertilité, des phénomènes inflammatoires, des maladies infectieuses ainsi qu'en tant que médicament pour la chimiothérapie anticancéreuse.

Selon la présente invention, le composé de formule (I) est tout particulièrement utile pour le traitement des troubles psychotiques, en particulier la schizophrénie ; pour le traitement des troubles de l'appétit et de l'obésité pour le traitement des troubles mnésiques et cognitifs ; pour le traitement de la dépendance alcoolique, de la dépendance nicotinique, c'est à dire pour le sevrage alcoolique et pour le sevrage tabagique.

Selon un de ses aspects, la présente invention est relative à l'utilisation d'un composé de formule (I) de ses sels pharmaceutiquement acceptables et de leurs solvats pour le traitement des troubles et maladies indiqués ci-dessus.

Selon un autre de ses aspects, la présente invention concerne également l'utilisation des composés de formule (I), tels quels ou sous forme radiomarquée comme outil pharmacologique chez l'homme ou chez l'animal, pour la détection et le marquage des récepteurs CB₁.

Le composé selon l'invention est généralement administré en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvats.

Le composé de formule (I) ci-dessus et ses sels ou solvats pharmaceutiquement acceptables peuvent être utilisés à des doses journalières de 0,01 à 100 mg par kg de poids corporel du mammifère à traiter, de préférence à des doses journalières de 0,02 à 50 mg/kg. Chez l'être humain, la dose peut varier de préférence de 0,05 à 4000 mg par jour, plus particulièrement de 0,1 à 1000 mg par jour selon l'âge du sujet à traiter ou le type de traitement, à savoir prophylactique ou curatif. Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,05 à 1000 mg, avantageusement de 0,1 à 500 mg, de préférence de 1 à 200 mg dudit principe actif par unité de dosage pour les administrations quotidiennes.

Lorsque l'on prépare une composition solide sous forme de comprimé, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Une préparation sous forme de sirop ou d'élixir peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion, des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intra-oculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le polyéthylèneglycol.

Ainsi, pour préparer une solution aqueuse injectable par voie intraveineuse, on peut utiliser un cosolvant comme par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades ou des gels.

Pour l'administration transdermique, on peut utiliser des patches sous forme multilaminée ou à réservoir dans lesquels le principe actif peut être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple a-, b- ou g-cyclodextrine, 2-hydroxypropyl-b-cyclodextrine ou méthyl-b-cyclodextrine.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Les compositions pharmaceutiques de la présente invention peuvent contenir, à côté du composé de formule (I) ou d'un de ses sels ou solvats pharmaceutiquement acceptable, d'autres principes actifs qui peuvent être utiles dans le traitement des troubles ou maladies indiquées ci-dessus.

Dans la présente description, on utilise les abréviations suivantes :
DCM : dichlorométhane
LiHMDS : sel de lithium de l'hexaméthyldisilazane
TMSCl : chlorotriméthylsilane
APTS : acide paratoluènesulfonique
NBS : N-bromosuccinimide
MTBE : méthyl-*tert*-butyléther
TA : température ambiante
F : point de fusion
CCM : chromatographie en couche mince
RMN : résonnance magnétique nucléaire. Les spectres RMN sont enregistrés à 200 MHz dans le DMSO d6
s: singulet ; d : doublet ; t : triplet ; q : quadruplet ; m : massif ou multiplet ; dd : doublet dédoublé.

### PREPARATION 1

Ester éthylique de l'acide 5-(4-bromophényl)-1-(2,4-dichlorophényl) -4-éthylpyrazole-3-carboxylique.

### A) Sel de lithium du 4-(4-bromophényl)-3-méthyl-4-oxydo-2-oxobutèn-oate d'éthyle.

Sous azote, on place 21,6 g de LiHMDS dans 340 ml d'éther anhydre et on refroidit à -60°C puis on ajoute 4 g de bromopropiophénone en solution dans 150 ml d'éther anhydre. On laisse remonter la température à -30°C puis on ajoute 17,53 ml d'oxalate d'éthyle. Après une nuit sous agitation à TA, le précipité formé est filtré puis rincé à l'éther et séché sous vide. On obtient 21,8 g du composé attendu.

### B) Ester éthylique de l'acide 4-(4-bromophényl)-2-[(2,4-dichlorophényl)-hydrazono]-3-méthyl-4-oxobutyrique.

On mélange 16,8 g du composé préparé à l'étape précédente et 12,5 g de chlorhydrate de 2,4-dichlorophénylhydrazine dans 150 ml d'éthanol et on laisse sous agitation pendant 2 heures et demie. Le précipité formé est filtré, rincé à l'éthanol puis séché sous vide. On obtient 16,24 g du composé attendu.

### C) Ester éthylique de l'acide 5-(4-bromophényl)-1-(2,4-dichlorophényl)-4-méthylpyrazole-3-carboxylique .

16,24 g du composé obtenu à l'étape précédente sont chauffés pendant 24 heures dans 200 ml d'acide acétique puis le milieu réactionnel est versé sur 1 litre d'eau glacée ; le précipité formé est filtré, rincé à l'eau et séché sous vide. On obtient 12,8 g du composé attendu qui est recristallisé dans le méthylcyclohexane, F = 133°C.

### D) Ester éthylique de l'acide 4-bromométhyl-5-(4-bromophényl)-1-(2,4-dichlorophényl)pyrazole-3-carboxylique.

On place 12,8 g d'ester obtenu à l'étape précédente dans 130 ml de tétrachlorure de carbone et on ajoute 5,27 g de N-bromosuccinimide puis 24 mg de péroxyde de benzoyle. Le mélange est chauffé 4 heures à reflux puis on filtre et concentre sous vide. Le résidu est chromatographié sur silice en éluant par un mélange toluène-acétate d'éthyle (97/3 ; v/v). On obtient 7,24 g du composé attendu, F = 116°C.

### E) Ester éthylique de l'acide 5-(4-bromophényl)-1-(2,4-dichlorophényl)-4-éthylpyrazole-3-carboxylique.

Sous argon, on introduit 2,26 g de CuBr en suspension dans 100 ml d'éther puis on ajoute goutte à goutte à -20°C une solution contenant 20 ml de méthyllithium 1,6 M dans l'éther dilué dans 20 ml d'éther. Après 10 minutes d'agitation à -20°C, la suspension se décolore puis devient limpide. On refroidit à -78°C et ajoute en 30 minutes 7 g du composé préparé à l'étape précédente, en solution dans 100 ml d'éther puis on laisse remonter à TA. Après 2 heures sous agitation, on hydrolyse par addition d'une solution saturée de chlorure d'ammonium. On extrait à l'éther, lave à l'eau puis par une solution saturée de NaCl. On sèche sur MgSO₄ puis évapore à sec. Le résidu est chromatographié sur silice en éluant par un mélange toluène-acétate d'éthyle (96/4 ; v/v). On obtient 3,7 g du composé attendu, F = 108°C. RMN : 1,05 ppm : t : 3H ; 1,30 ppm : t : 3H ; 2,60 ppm : q : 2H ; 4,30 ppm : q : 2H ; 7,15 ppm : d : 2H ; 7,50-7,75 ppm : m : 5H.

### PREPARATION 2

### Acide 5-(4-bromophényl)-1-(2,4-dichlorophényl)-4-éthylpyrazole-3-carboxylique (II).

3,6 g de l'ester obtenu à la préparation 1 sont placés dans 54 ml de MeOH et on ajoute une solution contenant 1,08 g de KOH dans 6,85 ml d'eau. Le milieu réactionnel est chauffé à reflux pendant 3 heures puis on concentre sous vide. Le résidu est repris par de l'eau glacée, acidifié à pH = 1 par HCl 1N puis extrait au DCM. On obtient 3,3 g du composé attendu, F = 218°C.

RMN : 1,10 ppm : t : 3H ; 2,70 ppm : q : 2H ; 7,25 ppm : d : 2H ;7,60-7,85 ppm : m : 5H.

### PREPARATION 3

Ester éthylique de l'acide 3-(4-bromobenzoyl)-2-oxopentanoïque.

On coule une solution de 247 g de 4-bromobutyrophénone dans 1500 ml de MTBE sur une solution de 210 g de LiHMDS dans 2500 ml de MTBE en maintenant la température à -20°C. Après 3 heures sous agitation à cette température, on ajoute en 1 heure, à 10°C, 210 g de l'ester éthylique de l'acide 2-(imidazol-1-yl)-2-oxoacétique dans 1000 ml de MTBE et on laisse sous agitation 18 heures à température ambiante. Le sel de lithium formé est filtré puis mis en suspension dans 800 ml de MTBE. On ajoute 800 ml d'acide chlorhydrique 6N à la suspension. Après décantation, la phase éthérée est lavée 4 fois par 1000 ml d'eau puis concentrée sous pression réduite. Le composé attendu est isolé (263 g). D'après l'analyse RMN, il est mélangé à 8 % de 4-bromobutyrophénone de départ.

RMN : 0,86 ppm : t : 3H ; 1,10 ppm : t : 3H ; 1,83 ppm : mt : 2H ; 4,15 ppm : q : 2H ; 5,19 ppm : t : 1H ; 7,70 ppm : d : 2H ; 7,98 ppm : d : 2H.

### PREPARATION 4

### Ester éthylique de l'acide 5-(4-bromophényl)-1-(2,4-dichlorophényl)-4-éthylpyrazole-3-carboxylique.

### A) Ester éthylique de l'acide 3-(4-bromobenzoyl)-2-(2-(2,4-dichlorophényl) hydrazono) pentanoate :.

On prépare une suspension de 155 g de chlorhydrate de 2,4-dichlorophényl hydrazine dans 1200 ml d'éthanol et on ajoute à température ambiante 263 g du composé de la préparation 3 dans 1000 ml d'éthanol.

On peut isoler par filtration une petite partie de l'intermédiaire formé et le caractériser.

RMN : 0,92 ppm : t : 3H ; 1,04 ppm : t : 3H ; 1,89 ppm : mt : 2H ; 4,16 ppm : q : 2H ; 4,76 ppm : t : 1H ; 7,42 ppm : mt : 2H ; 7,60 ppm : s : 1H ; 7,75 ppm : d : 2H ; 7,93 ppm : d : 2H ; 12,31 ppm : s : 1H.

### B) Ester éthylique de l'acide 5-(4-bromophényl)-1-(2,4-dichlorophényl) -4-éthylpyrazole-3-carboxylique.

La suspension obtenue est portée au reflux pendant 4 heures puis laissé 18 heures sous agitation à température ambiante. On filtre le produit formé puis on le sèche sous vide à 50°C pour obtenir le composé attendu (247 g), F = 108°C.

RMN : 1,07 ppm : t : 3H ; 1,28 ppm : t : 3H ; 2,58 ppm : q : 2H ; 4,32 ppm : q : 2H ; 7,16 ppm : d : 2H ; 7,53 ppm : dd : 1H ; 7,59 ppm : d : 2H ; 7,73 ppm : d + petit d : 2H.

### EXEMPLE 1

### N-pipéridino-5-(4-bromophényl)-1-(2,4-dichlorophényl)-4-éthylpyrazole-3-carboxamide.

### A) Chlorure de l'acide 5-(4-bromophényl)-1-(2,4-dichlorophényl)-4-éthylpyrazole-3-carboxylique.

On place 3,2 g de l'acide obtenu à l'étape précédente en suspension dans 32 ml de toluène, on ajoute 1,6 ml de chlorure de thionyle puis on chauffe à reflux pendant 3 heures. Le milieu réactionnel est concentré sous vide puis repris par du toluène. L'opération est répétée plusieurs fois. On obtient 3,3 g du composé attendu.

### B) N-pipéridino-5-(4-bromophényl)-1-(2,4-dichlorophényl)-4-éthylpyrazole-3-carboxamide.

Sous azote, on prépare une solution de 0,23 ml de N-aminopipéridine et 0,29 ml de triéthylamine dans 20 ml de DCM et on refroidit à une température comprise entre 0° et 5°C. On ajoute 0,8 g du chlorure d'acide obtenu à l'étape précédente dans 20 ml de DCM. Après une nuit à TA, on coule sur de l'eau glacée et décante. La phase organique est extraite au DCM puis lavée à l'eau, par une solution de Na₂CO₃ à 5 %, par une solution saturée de NaCl. On évapore à sec puis le résidu est chromatographié sur silice en éluant par un mélange toluène/AcOEt (80/20 ; v/v). On obtient 0,52 g du composé attendu, F = 113°C.

RMN : 1,05 ppm : t : 3H ; 1,25-1,65 ppm : m : 6H ; 2,65 ppm : q : 2H ; 2,80 ppm : m : 4H ; 7,15 ppm : d : 2H ; 7,50-7,80 ppm : m : 5H ; 9,10 ppm : s : 1H.

### EXEMPLE 2

### N-pipéridino-5-(4-bromophényl)-1-(2,4-dichlorophényl)-4-éthylpyrazole-3-carboxamide.

### A) Chlorure de l'acide 5-(4-bromophényl)-1-(2,4-dichlorophényl)-4-éthylpyrazole-3-carboxylique.

On prépare un mélange contenant 97 g de chlorure de thionyle et 118 g du composé de la préparation 4 dans 1200 ml de toluène et l'on chauffe progressivement jusqu'au reflux, puis on maintient le reflux pendant 3 heures. Le milieu réactionnel est concentré.

### B) N-pipéridino-5-(4-bromophényl)-1-(2,4-dichlorophényl)-4-éthylpyrazole-3-carboxamide.

Le chlorure d'acide formé est repris par 380 ml de méthylcyclohexane et on introduit 2,8 g de triéthylamine dans 218 ml de THF. Le mélange est maintenu à 50°C.

On prépare une solution de 30 g de N-aminopipéridine et 28 g de triéthylamine dans 34 ml de méthylcyclohexane que l'on refroidit à 10°C et l'on ajoute lentement le mélange contenant le chlorure d'acide. Après 2 heures sous agitation à 10°C, on filtre le produit formé, on le reprend par 2000 ml de DCM et on le lave 2 fois par 2000 ml d'eau. Le produit est recristallisé dans 4500 ml de méthylcyclohexane puis filtré et séché. On obtient 125 g du composé attendu.

## Revendications

1. Le N-pipéridino-5-(4-bromophényl)-1-(2,4-dichloro phényl)-4-éthylpyrazole-3-carboxamide de formule : 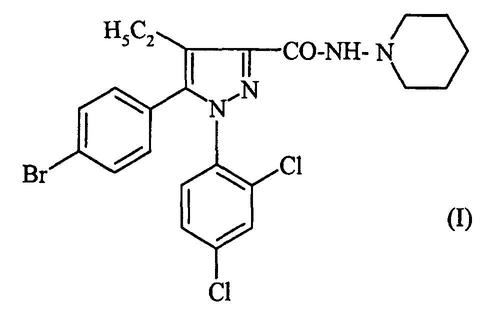 ses sels pharmaceutiquement acceptables et leurs solvats.

2. Procédé pour la préparation du N-pipéridino-5-(4-bromophényl)-1-(2,4-dichlorophényl)-4-éthylpyrazole-3-carboxamide, de ses sels et de leurs solvats, **caractérisé en ce qu'**on traite un dérivé fonctionnel de l'acide 5-(4-bromophényl)-1-(2,4-dichlorophényl)-4-éthylpyrazole-3-carboxylique de formule : 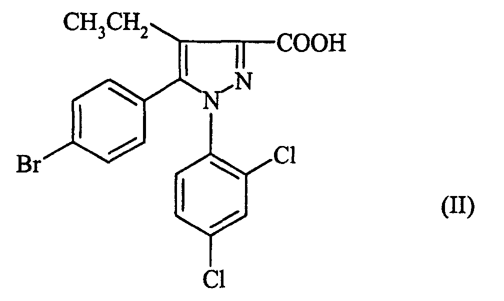 avec la 1-aminopipéridine, dans un solvant organique et en présence d'une base ; et éventuellement on transforme le composé ainsi obtenu en l'un de ses sels ou l'un de leurs solvats.

3. Procédé pour la préparation d'un ester alkylique de l'acide 5-(4-bromophényl)-1-(2,4-dichlorophényl)-4-éthylpyrazole-3-carboxylique par cyclisation d'un ester alkylique de l'acide 3-(4-bromobenzoyl)-2-(2-(2,4-dichlorophényl)hydrazono) pentanoate (IX).

4. Procédé pour la préparation d'un ester alkylique de l'acide 3-(4-bromobenzoyl)-2-(2-(2,4-dichlorophényl)hydrazono)pentanoate par action d'un sel de 2,4-dichlorophénylhydrazine sur un ester alkylique de l'acide 4-bromobenzoyl-2-oxopentanoique (VIII).

5. Procédé pour la préparation d'un ester alkylique de l'acide 4-bromobenzoyl-2-oxopentanoïque par action de LiHMDS puis d'un ester alkylique de l'acide 2-(imidazol-1-yl)-2-oxoacétique sur la bromobutyrophénone.

6. Procédé pour la préparation d'un ester alkylique de l'acide 5-(4-bromophényl)-1-(2,4-dichlorophényl)-4-éthylpyrazole-3-carboxylique à partir de l'acide 4-bromobenzoyl-2-oxopentanoïque par action d'un sel de 2,4-dichlorophénylhydrazine puis cyclisation.

7. Le composé de formule : 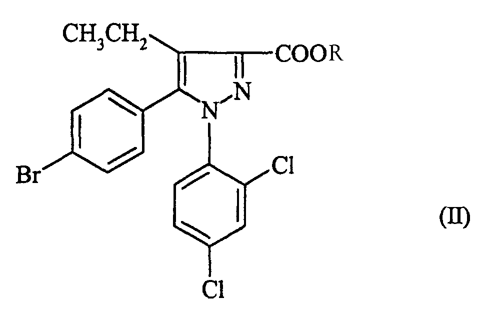 dans leguelle R repésente H ou (C₁-C₄) alkyle.

8. Un composé selon la revendication 7 de formule : 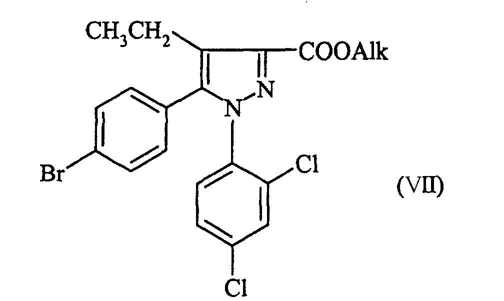 dans laquelle Alk représente un (C₁-C₄)alkyle.

9. Un composé de formule : 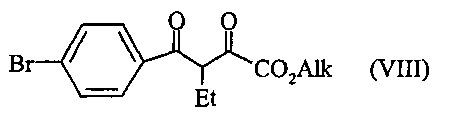 dans laquelle Alk représente un (C₁-C₆)alkyle.

10. Un composé de formule : 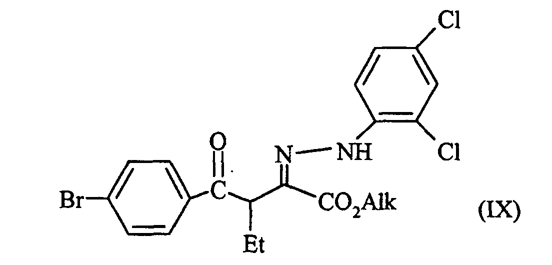 dans laquelle Alk représente un (C₁-C₆)alkyle.

11. Composition pharmaceutique contenant, en tant que principe actif, un composé selon la revendication 1.

12. Composition pharmaceutique selon la revendication 11, contenant de 0,1 à 1000 mg de principe actif, sous forme d'unité de dosage, dans laquelle le principe actif est mélangé à au moins un excipient pharmaceutique.

13. Utilisation d'un composé selon la revendication 1 pour la préparation de médicaments destinés à traiter les maladies impliquant les récepteurs aux cannabinoides CB₁.

14. Utilisation d'un composé selon la revendication 13 pour le traitement des troubles psychotiques, pour le traitement des troubles de l'appétit et de l'obésité, pour le traitement des troubles mnésiques et cognitifs ; pour le traitement de la dépendance alcoolique et pour le sevrage tabagique.

## Patentansprüche

1. N-Piperidino-5-(4-bromphenyl)-1-(2,4-dichlorphenyl)-4-ethylpyrazol-3-carboxamid der Formel: 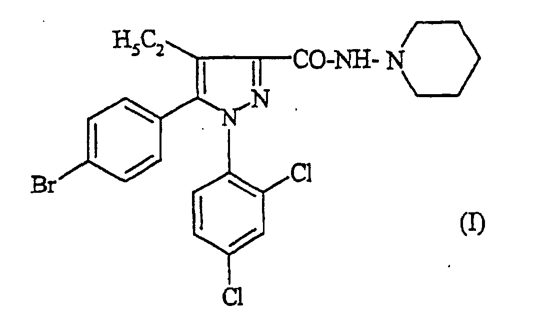 dessen pharmazeutisch annehmbaren Salze und dessen Solvate.

2. Verfahren zur Herstellung von N-Piperidino-5-(4-bromphenyl)-1-(2,4-dichlorphenyl)-4-ethylpyrazol-3-carboxamid, von dessen Salzen und dessen Solvaten, **dadurch gekennzeichnet, daß** man ein funktionelles Derivat der 5-(4-Bromphenyl)-1-(2,4-dichlorphenyl)-4-ethylpyrazol-3-carbonsäure der Formel: 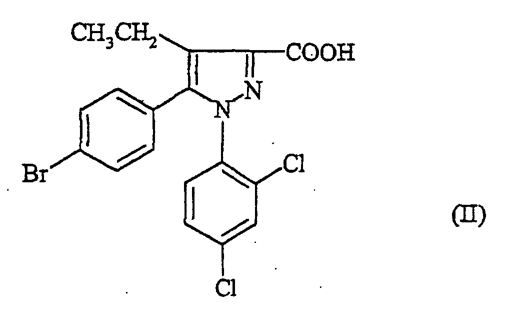 in einem organischen Lösungsmittel und in Gegenwart einer Base mit 1-Aminopiperidin behandelt und gegebenenfalls die in dieser Weise erhaltene Verbindung in eines ihrer Salze oder eines ihrer Solvate umwandelt.

3. Verfahren zur Herstellung eines Alkylesters der 5-(4-Bromphenyl)-1-(2,4-dichlorphenyl)-4-ethylpyrazol-3-carbonsäure durch Cyclisieren eines 3-(4-Brombenzoyl)-2-(2-(2,4-dichlorphenyl)-hydrazono)-pentansäurealkylesters (IX).

4. Verfahren zur Herstellung eines 3-(4-Brombenzoyl)-2-(2-(2,4-dichlorphenyl)-hydrazono)-pentansäurealkylesters durch Umsetzen eines 2,4-Dichlorphenylhydrazinsalzes mit einem 4-Brombenzoyl-2-oxopentansäurealkylester (VIII).

5. Verfahren zur Herstellung eines Alkylesters der 4-Brombenzoyl-2-oxopentansäure durch Einwirkung von LiHMDS und dann eines Alkylesters der 2-(Imidazol-1-yl)-2-oxoessigsäure auf Brombutyrophenon.

6. Verfahren zur Herstellung eines Alkylesters der 5-(4-Bromphenyl)-1-(2,4-dichlorphenyl)-4-ethylpyrazol-3-carbonsäure ausgehend von 4-Brombenzoyl-2-oxopentansäure durch Einwirkung eines 2,4-Dichlorphenylhydrazinsalzes und anschließende Cyclisierung.

7. Verbindung der Formel: 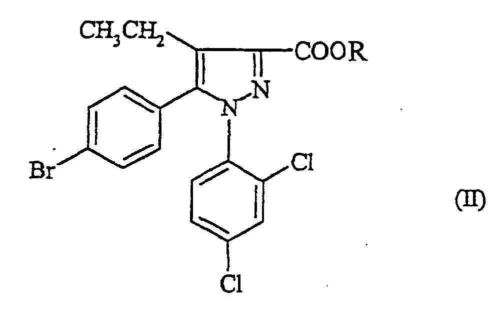 in der R H oder (C₁-C₄)-Alkyl bedeutet.

8. Verbindung nach Anspruch 7 der Formel: 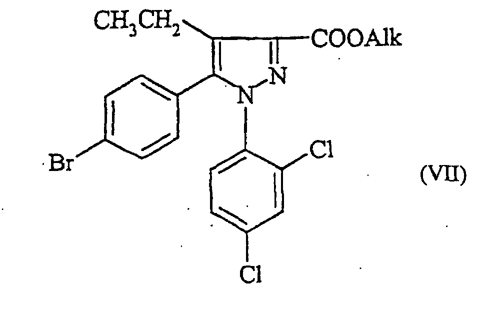 in der Alk eine (C₁-C₄)-Alkylgruppe bedeutet.

9. Verbindung der Formel: 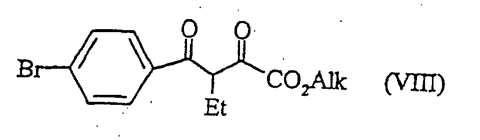 in der Alk eine (C₁-C₆)-Alkylgruppe darstellt.

10. Verbindung der Formel: 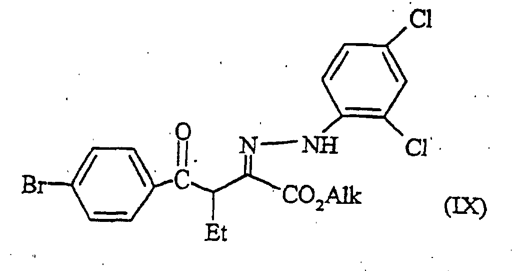 in der Alk eine (C₁-C₆)-Alkylgruppe darstellt.

11. Pharmazeutische Zubereitung enthaltend als Wirkstoff eine Verbindung nach Anspruch 1.

12. Pharmazeutische Zubereitung nach Anspruch 11, enthaltend 0,1 bis 1000 mg des Wirkstoffes in Einheitsdosisform, in der der Wirkstoff mit mindestens einem pharmazeutischen Trägermaterial vermischt ist.

13. Verwendung einer Verbindung nach Anspruch 1 für die Herstellung von Arzneimitteln zur Behandlung von Erkrankungen, bei denen die Cannabinoid-Rezeptoren CB₁ beteiligt sind.

14. Verwendung einer Verbindung nach Anspruch 13 für die Behandlung von psychotischen Störungen, zur Behandlung von Appetitstörungen und der Fettsucht, zur Behandlung von Gedächtnis- und Erkenntnis-Störungen, für die Behandlung der Alkoholabhängigkeit und für den Tabakentzug.

## Claims

1. N-Piperidino-5-(4-bromophenyl)-1-(2,4-dichlorophenyl)-4-ethylpyrazole-3-carboxamide, of formula: 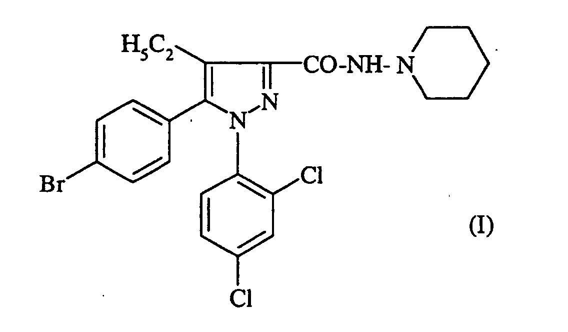 its pharmaceutically acceptable salts and the solvates thereof.

2. Process for preparing N-piperidino-5-(4-bromophenyl)-1-(2,4-dichlorophenyl)-4-ethylpyrazole-3-carboxamide, its salts and the solvates thereof, **characterized in that** a functional derivative of 5-(4-bromophenyl)-1-(2,4-dichlorophenyl)-4-ethylpyrazole-3-carboxylic acid, of formula: 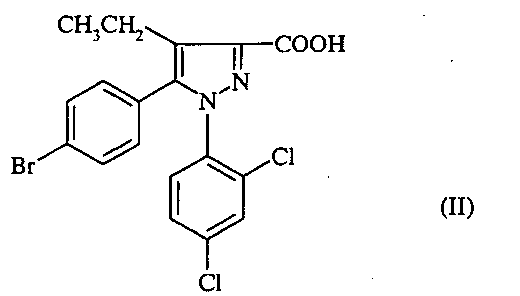 is treated with 1-aminopiperidine, in an organic solvent and in the presence of a base; and the compound thus obtained is optionally converted into one of its salts or one of the solvates thereof.

3. Process for preparing an alkyl ester of 5-(4-bromophenyl)-1-(2,4-dichlorophenyl)-4-ethylpyrazole-3-carboxylic acid by cyclization of an alkyl ester of 3-(4-bromobenzoyl)-2-(2-(2,4-dichlorophenyl)hydrazono)pentanoic acid (IX).

4. Process for preparing an alkyl ester of 3-(4-bromobenzoyl)-2-(2-(2,4-dichlorophenyl)hydrazono)pentanoic acid by the action of a 2,4-dichlorophenylhydrazine salt on an alkyl ester of 4-bromobenzoyl-2-oxopentanoic acid (VIII).

5. Process for preparing an alkyl ester of 4-bromobenzoyl-2-oxopentanoic acid by the action of LiHMDS and then an alkyl ester of 2-(1-imidazolyl)-2-oxoacetic acid on bromobutyrophenone.

6. Process for preparing an alkyl ester of 5-(4-bromophenyl)-1-(2,4-dichlorophenyl)-4-ethylpyrazole-3-carboxylic acid from 4-bromobenzoyl-2-oxopentanoic acid by the action of a 2,4-dichlorophenylhydrazine salt, followed by cyclization.

7. Compound of formula: 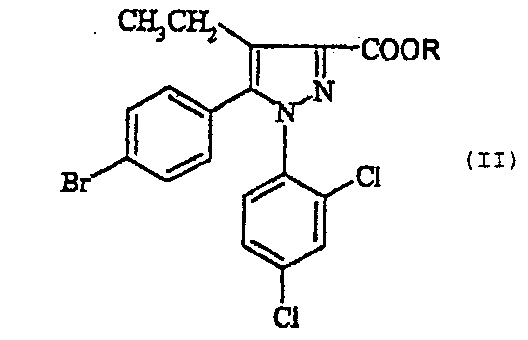 in which R represents H or (C₁-C₄) alkyl.

8. Compound according to Claim 7, of formula: 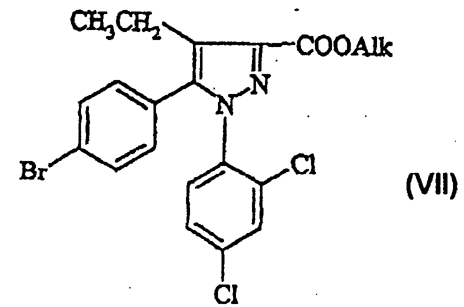 in which Alk represents a (C₁-C₄) alkyl.

9. Compound of formula: 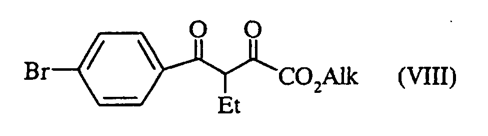 in which Alk represents a (C₁-C₆)alkyl.

10. Compound of formula 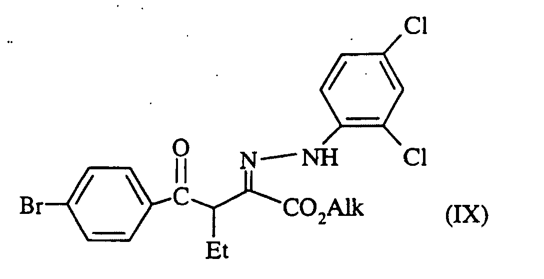 in which Alk represents a (C₁-C₆)alkyl.

11. Pharmaceutical composition containing, as active principle, a compound according to Claim 1.

12. Pharmaceutical composition according to Claim 11, containing from 0.1 to 1000 mg of active principle, in unit dosage form, in which the active principle is mixed with at least one pharmaceutical excipient.

13. Use of a compound according to Claim 1 for the preparation of medicinal products intended for treating diseases involving the CB₁ cannabinoid receptors.

14. Use of a compound according to Claim 13 for the treatment of psychotic disorders, for the treatment of appetite disorders and obesity, for the treatment of memory and cognitive disorders; for the treatment of alcohol dependency and for withdrawal from tobacco.
